# EUROPÄISCHE PATENTSCHRIFT

(11) **EP 1 621 153 B1**
(45) Veröffentlichungstag und Bekanntmachung des Hinweises auf die Patenterteilung: **15.08.2007**
(21) Anmeldenummer: 04017859.2
(22) Anmeldetag: 28.07.2004
(51) Int. Cl.: A61B 19/00, H04N 13/00

(54) **Stereoskopische Visualisierungsvorrichtung für Patientenbilddaten und Videobilder**
Stereoscopic visualisation apparatus for the combination of scanned and video images
Appareil de visualisation stéréoscopique de données d'imagerie médicale et d'images vidéo combinées

(43) Veröffentlichungstag der Anmeldung: 01.02.2006
(73) Patentinhaber: BrainLAB AG, 85551 Kirchheim/Heimstetten (DE)
(72) Erfinder: Birkenbach, Rainer, 85445 Aufkirchen (DE)
(74) Vertreter: Schwabe - Sandmair - Marx

(56) Entgegenhaltungen:
- EP-A- 0 877 274
- EP-B- 1 321 105
- WO-A-00/36845
- WO-A-03/002011
- US-A- 5 978 143
- US-A1- 2002 082 498

## Beschreibung

Die vorliegende Erfindung betrifft eine Vorrichtung zur visuellen Kombination von Patientenbilddaten aus Durchleuchtungs- bzw. Schichtbilderfassungsverfahren bzw. Objektbilddaten mit Videobildern, die als tragbare Einheit ausgebildet ist. Die Vorrichtung umfasst eine Bildanzeigevorrichtung, eine Kameravorrichtung und ein computergestütztes Navigationssystem, dass die Raumpositionen der Bildanzeigevorrichtung und/oder der Kameravorrichtung sowie die Rahmenpositionen eines Patientenkörperteils über dort angebrachte Trackingeinrichtungen erfassen kann.

Eine solche Vorrichtung ist in der EP 1 321 105 B1 beschrieben. Dort wird eine tragbare Bildschirm-Kamera-Einheit mit einer Kamera auf der Rückseite des Bildschirms offenbart. Der Arzt kann eine solche Bildanzeigevorrichtung mit zugeordneter Kamera vor ein Patientenkörperteil halten und durch die kombinierte Darstellung von Patientenbilddaten aus Durchleuchtungs- bzw. Schichtbilderfassungsverfahren und Videobildern das Patientenkörperteil von außen ansehen und gleichzeitig überlagerte Bilder für die Strukturen im Inneren des Körperteils erhalten.

Nachteilig ist dabei, dass dem Arzt bzw. jedem Betrachter lediglich eine flache Projektion, d.h. eine zweidimensionale Ansicht geliefert wird.

Es ist die Aufgabe der vorliegenden Erfindung, eine solche Vorrichtung dahingehend zu verbessern, dass dem Betrachter mehr Informationen geliefert werden, als dies mit einer flachen Projektion möglich ist.

Diese Aufgabe wird erfindungsgemäß dadurch gelöst, dass die Bildanzeigevorrichtung mit einem autostereoskopischen Monitor ausgerüstet wird. Autostereoskopische Monitor sind z.B. aus der US 5,978,143 bekannt.

Mit anderen Worten wird erfindungsgemäß die zweidimensionale Darstellung der Bildanzeigevorrichtung auf eine dreidimensionale Darstellung erweitert. Dabei macht die erfindungsgemäße Vorrichtung es sich zu Nutze, dass die Patientenbilddaten, als Bilddaten, die beispielsweise aus Durchleuchtungs- oder Schichtbilderfassungsverfahren stammen, in vielen Fällen schon als räumliche und dreidimensionale Daten zur Verfügung stehen. Wenn beispielsweise eine CT-Aufnahme gemacht wird, stehen aus den Schichtbildern schon dreidimensionale oder räumliche Patientenbilddaten bereit. Dieser Umstand wurde bei der bisher bekannten Vorrichtung nur in soweit ausgenutzt, dass man die dargestellten Körperteil-Strukturmerkmale immer richtig in Blickrichtung projiziert darstellen konnte, aber immer zweidimensional auf dem Bildschirm.

Mit der vorliegenden Erfindung wird es nunmehr möglich, den gesamten Informationsgehalt der dreidimensionalen Patientenbilddatensätze auch visuell zur Verfügung zu stellen. Dazu wird ein autostereoskopischer Monitor verwendet, der dem Betrachter ein dreidimensionales Bild liefert, ohne dass zusätzliche Hilfsmittel, wie z.B. Brillen, notwendig werden. Je nach Bauart des autostereoskopischen Monitors können diesem die Patientenbilddaten in geeigneter Weise so bereit gestellt werden, dass sie für den Betrachter dreidimensional erscheinen.

Wenn gemäß einer bevorzugten Ausführungsform der Erfindung die Kameraeinrichtung eine stereoskopische Kameraeinrichtung ist, lassen sich auch die von dieser Kameraeinrichtung erfassten Daten für das Äußere des Patienten so bearbeiten, dass der Bildschirm, dem sie bereitgestellt werden, ein stereoskopisches bzw. dreidimensionales Videobild des Patientenkörperteils von außen erzeugt. Weil die Daten mittels des Navigationssystems positionell zugeordnet werden können, lassen sich beide Bilder so überlagern, dass eine dreidimensionale Ansicht auf dem Monitor entsteht, die es dem Betrachter gestattet, das Patientenkörperteil gleichzeitig anhand von äußeren und inneren Merkmalen zu betrachten und dabei auch Tiefeninformationen zu erhalten.

Autostereoskopische Monitore stehen zur Verfügung und können an die Bedürfnisse der erfindungsgemäßen Anwendung angepasst werden. Es gibt sogenannte Parallaxen-Displays, die auf einer zweidimensionalen Bildebene und einer aktiven Bündelung und Ausrichtung des diffusen Lichtes beruhen. Sogenannte Parallaxen-Barrier-Systeme verwenden eine Art Lochmaske, also eine lichtundurchlässige Schicht vor der Bildfläche, die durch regelmäßige Schlitze unterbrochen ist, wobei ein definierter Bildbereich abhängig vom Blickwinkel präsentiert wird. Andere Systeme, die wie die Parallaxen-Systeme mit der vorliegenden Erfindung zur Anwendung kommen können, sind Linsen basierte Systeme bei denen die Ansichten durch Linsenelemente vor dem Bildschirm getrennt werden. Es können runde Linsen verwendet werden (Vollparallaxe), oder halbzylindrische optische Linsen, die bei schräger Ausrichtung eine bessere Auflösung gestatten und eine Sub-Pixel-Ansteuerung verwenden (slanted lenticular sheets). Schräge Sub-Pixel-Anordnungen vermeiden Moiré-Effekte und schwarze Übergänge. Auch die Verwendung von alternativen Parallaxen-Systemen ist möglich, z.B. von Prismen-Systemen oder polarisierenden Filtern.

Die Kameraeinrichtung kann eine einzige Kamera umfassen. Bei einer vorteilhaften Ausführungsform der vorliegenden Erfindung umfasst die Kameraeinrichtung mindestens zwei Kameras, die auf der dem Bildschirm entgegengesetzten Rückseite der Vorrichtung angeordnet sind und deren Abstand zueinander abgestimmt ist auf einen vorgegebenen Abstandsbereich zwischen Betrachter und Monitor. Die Kameraeinrichtung kann auch bewegliche Kameras aufweisen, um den Schnittbereich der Kamera-Sichtfelder in Abstimmung auf den Abstand der Kameras zum betrachteten Objekt und/oder in Abstimmung auf den Betrachter und Monitor einzustellen. So lässt sich die gesamte Anordnung, aber auch die verwendete Software beispielsweise vorab schon auf ein typisches Szenario einstellen, in dem entsprechende Vorgabewerte oder Wertebereiche festgelegt und verwendet werden. Damit lässt sich beispielsweise das Bild-Rendering vereinfachen und rechnerisch schneller gestalten. Durch die beweglichen Kameras erhöht sich die Flexibilität bezüglich des Abstandes zwischen der tragbaren Bildanzeigevorrichtung und des Patienten.

Der Monitor kann durch eine Bildverarbeitungseinheit angesteuert werden, welche die stereoskopischen Videobilder und dreidimensional berechneten Patienten-Körperstrukturen aus den Durchleuchtungs- bzw. Schichtbildern räumlich zuordnet und kombiniert, wobei ein kombiniertes Stereobild erzeugt wird. Der Monitor kann, wie oben schon angedeutet, ein Lochmasken- oder ein Linsenmonitor mit einer Stereo-Betrachtungszone oder mit mehreren Stereo-Betrachtungszonen sein. Es lassen sich also Single-User- und Multi-User-Konfigurationen einsetzen. Dabei kann gemäß einer Ausführungsform die Betrachtungszone oder jede Betrachtungszone stationär gegenüber dem Monitor angeordnet sein, d.h. es wird ein sogenanntes passives System bereitgestellt, bei dem der Betrachter ein gutes stereoskopisches Bild sieht, wenn er in einer oder in mehreren vorbestimmten Betrachtungszonen vor dem Monitor steht.

Es ist im Rahmen der vorliegenden Erfindung aber auch denkbar und vorteilhaft, dem Monitor eine Betrachter-Trackingeinheit zuzuordnen und damit ein sogenanntes aktives System zu schaffen, das die Betrachtungszone(n) dem Betrachter nachführt, indem durch die Bildverarbeitungseinheit auf dem Monitor ein Bild angezeigt wird, das seine Betrachtungszone(n) an dem oder jedem getrackten Betrachterstandort entwickelt. Ein solches "aktives" System stellt also fest, wo der Betrachter ist und liefert ihm an diesem Standort gerade das beste stereoskopische Bild.

Die Betrachter-Trackingeinheit kann einerseits eine Trackingeinheit des Navigationssystems sein oder diesem unmittelbar zugeordnet sein. Bei einer anderen Ausführungsform ist die Betracher-Trackingeinheit eine Video-Trackingeinheit mit einer separaten Videokamera, die mittels Bilderkennung die Position des Kopfes oder der Augen des Betrachters identifiziert und verfolgt.

Es besteht die Möglichkeit, als Bildverarbeitungseinheit diejenige des Navigationssystems zu verwenden oder eine Bildverarbeitungseinheit, die dem Navigationssystem unmittelbar zugeordnet ist, z.B. eine Bildverarbeitungseinheit in der durch das Navigationssystem getrackten, tragbaren Bildschirmeinheit. Natürlich kann die Bildverarbeitung auch separat durch eine separate Bildverarbeitungseinheit durchgeführt werden, wobei die Daten dann per Kabel oder kabellos zum Monitor übertragen werden.

Gemäß einer weiteren Ausführungsform der vorliegenden Erfindung weist die Vorrichtung noch eine Benutzer-Eingabevorrichtung auf, mittels der insbesondere:
- zwischen stereoskopischer und Normalbild-Betrachtungsweise umgeschaltet werden kann; und/oder
- zwischen der Darstellung mit einer oder mehreren Betrachtungszonen umgeschaltet werden kann; und/oder
- Eingaben für eine bildunterstützte Behandlung oder Behandlungsplanung ermöglicht werden; und/oder
- bewegliche Kameras angesteuert werden.

Die Erfindung wird im Weiteren anhand einer Ausführungsform näher erläutert, wobei auf die beiliegenden Figuren Bezug genommen wird. Die Erfindung kann mit allen ihren beschriebenen Merkmalen einzeln oder in Kombination umgesetzt werden, und sie umfasst auch die verfahrensmäßige Ausgestaltung der anhand der Vorrichtung beschriebenen Merkmale. In den Zeichnungen zeigen:
- Figur 1: eine erfindungsgemäße Vorrichtung, ausgestaltet als Bildschirm-Kameraeinheit, von der Vorderseite;
- Figur 2: eine Rückansicht einer Bildschirm-Kameraeinheit mit zwei rückseitigen Kameras; und
- Figur 3: eine Rückansicht einer Bildschirm-Kameraeinheit mit einer rückseitigen Kamera.

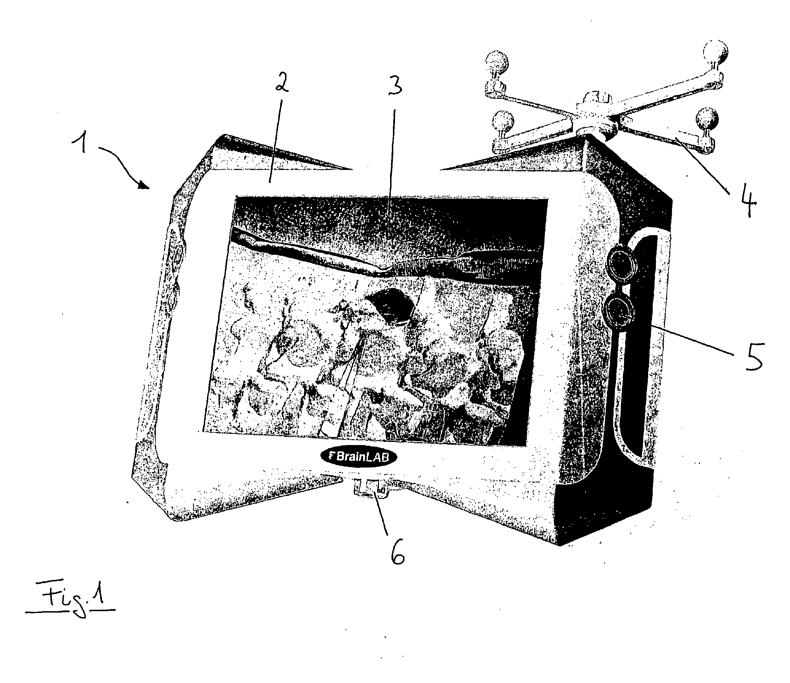

Die in Figur 1 von vorne perspektivisch dargestellte Bildanzeigeeinheit trägt insgesamt das Bezugszeichen 1. Sie weist ein Gehäuse 2 auf, das an seiner Vorderseite mit dem Bildschirm 3 bestückt ist. Der Bildschirm 3 ist ein autostereoskopischer Bildschirm bzw. Monitor, und im vorliegenden Fall ist auf seiner Anzeige ein teilweise abgedeckter Patientenrücken mit darunter liegender Wirbelsäule dargestellt.

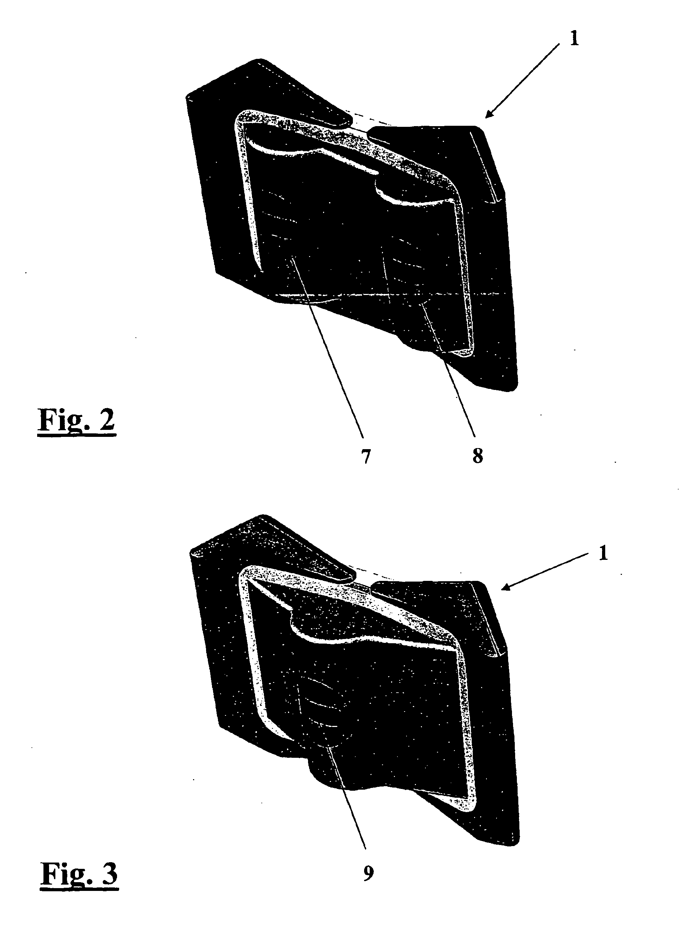

Oben am Gehäuse 2 ist ein Referenzstern 4 mit Markern gezeigt, der es möglich macht, die Position der Einheit 1 im Ortungsraum eines Navigationssystems eindeutig festzustellen, damit auch die Position der in Figur 2 gezeigten Kameras 7 und 8 auf der Rückseite, wodurch schlussendlich auch das Videobild dieser Kamera räumlich im Navigationssystem zugeordnet und in richtigem räumlichen Positionsverhältnis zu den inneren Patientenstrukturen dargestellt werden kann, da auch der Patient durch das Navigationssystem getrackt wird. Weil die beiden Kameras dazu in der Lage sind ein stereoskopisches Bild zu erzeugen und der Monitor ein autostereoskopischer Monitor ist, und weil zusätzlich die Daten über die innere Patientenstruktur (hier z.B. die Wirbelsäule) als räumliche Daten vorliegen (z.B. aus einer CT-Aufnahme) können dann alle Bildinformationen in einem einzigen kombinierten und überlagerten Stereobild so wiedergegeben werden, dass bei dem Betrachter die Bildtiefenwirkung entsteht. Im vorliegenden Fall ist die Trackingeinrichtung, also der Referenzstern 4, eine optische Trackingeinrichtung für das Navigationssystem, welche beispielsweise zwei beabstandete Infrarotkameras und einen Infrarot-Lichtabstrahler aufweist. Die Verwendung anderer Navigations- bzw. Trackingsysteme ist möglich (magnetisch oder aktiv abstrahlende Marker- oder Referenzanordnungen).

Der Betrachter hat nun den Vorteil, ein dreidimensionales Abbild betrachten zu können und sozusagen in den Patienten hineinzusehen; es entsteht ein Stereo-Bild bzw. ein dreidimensionales Bild eines "gläsernen Patienten".

An der Einheit 1 ist noch im unteren Teil des Gehäuses 2 eine Befestigungsvorrichtung 6 angebracht. Mit Hilfe dieser Vorrichtung kann der Monitor, der als tragbare Einheit ausgebildet ist, vorübergehend oder dann, wenn längere Zeit aus derselben Richtung auf und in den Patienten gesehen werden soll, an einer nicht dargestellten Halterung befestigt werden. Ferner weist der Bildschirm noch Eingabeknöpfe auf, von denen einer rechts am Gehäuse mit dem Bezugszeichen 5 versehen wurde. Mit Hilfe dieser Eingabeknöpfe (es kann auch ein Touchscreen oder eine andere Eingabevorrichtung verwendet werden) kann beispielsweise zwischen stereoskopischer und Normalbild-Betrachtungsweise umgeschaltet werden. Ferner besteht die Möglichkeit, zwischen der Darstellung mit einer oder mehreren Betrachtungszonen umzuschalten und/oder Eingaben für eine bildunterstützte Behandlung oder Behandlungsplanung zu ermöglichen. Wenn die Kameras 7, 8 auf der Rückseite als bewegliche Kameras ausgebildet werden, um den Sichtfeld-Bereich einstellbar zu machen, kann auch die Bewegung der Kamera mit Hilfe der Einstellknöpfe 5 gesteuert werden. Hierzu empfiehlt es sich, dass über die Knöpfe eine Menüsteuerung verfügbar gemacht wird.

## Patentansprüche

1. Vorrichtung zur visuellen Kombination von Patientenbilddaten aus Durchleuchtungs- bzw. Schichtbilderfassungsverfahren bzw. Objektbilddaten mit Videobildern, mit einer Bildanzeigevorrichtung (1), einer Kameraeinrichtung (7, 8) und einem computergestützten Navigationssystem, das die Raumpositionen der Bildanzeigevorrichtung (1) und/oder der Kameravorrichtung (7, 8) sowie die Raumpositionen eines Patientenkörperteils über dort angebrachte Trackingeinrichtungen (4) erfassen kann, wobei die Bildanzeigevorrichtung (1) und die Kameravorrichtung (7, 8) einander zugeordnet und als tragbare Einheit ausgebildet sind, **dadurch gekennzeichnet, dass** die Bildanzeigevorrichtung (1) einen autostereoskopischen Monitor (3) aufweist.

2. Vorrichtung nach Anspruch 1, **dadurch gekennzeichnet, dass** die Kameraeinrichtung eine stereoskopische Kameraeinrichtung (7, 8) ist.

3. Vorrichtung nach Anspruch 1 oder 2, **dadurch gekennzeichnet, dass** die Kameraeinrichtung mindestens zwei Kameras (7, 8) umfasst, die auf der dem Monitor (3) entgegengesetzten Rückseite der Vorrichtung (1) angeordnet sind und deren Abstand zueinander abgestimmt ist auf einen vorgegebenen Abstandsbereich zwischen Betrachter und Monitor (3).

4. Vorrichtung nach Anspruch 3, **dadurch gekennzeichnet, dass** die Kameraeinrichtung bewegliche Kameras (7, 8) aufweist, um den Schnittbereich der Kamera-Sichtfelder in Abstimmung auf den Abstand der Kameras zum betrachteten Objekt und/oder in Abstimmung auf den Abstand zwischen Betrachter und Monitor (3) einzustellen.

5. Vorrichtung nach einem der Ansprüche 1 bis 4, **dadurch gekennzeichnet, dass** der Monitor (3) durch eine Bildverarbeitungseinheit angesteuert wird, welche die stereoskopischen Videobilder und dreidimensional berechneten Patienten-Körperstrukturen aus den Durchleuchtungs- bzw. Schichtbildern räumlich zuordnet und kombiniert, wobei ein kombiniertes Stereobild angezeigt wird.

6. Vorrichtung nach einem der Ansprüche 1 bis 5, **dadurch gekennzeichnet, dass** der Monitor (3) ein Lochmasken- oder ein Linsenmonitor mit einer Stereo-Betrachtungszone oder mit mehreren Stereo-Betrachtungszonen ist.

7. Vorrichtung nach Anspruch 6, **dadurch gekennzeichnet, dass** die Betrachtungszone(n) stationär gegenüber dem Monitor (3) angeordnet sind.

8. Vorrichtung nach Anspruch 6, **dadurch gekennzeichnet, dass** dem Monitor (3) eine Betrachter-Trackingeinheit zugeordnet ist und die Betrachtungszone(n) dem Betrachter nachgeführt wird, indem durch die Bildverarbeitungseinheit auf dem Monitor (3) ein Bild angezeigt wird, das seine Betrachtungszone(n) an dem oder jedem getrackten Betrachterstandort entwickelt.

9. Vorrichtung nach Anspruch 8, **dadurch gekennzeichnet, dass** die Betrachter-Trackingeinheit eine Trackingeinheit des Navigationssystems ist oder dieser unmittelbar zugeordnet ist.

10. Vorrichtung nach einem der Ansprüche 4 bis 8, **dadurch gekennzeichnet, dass** die Betrachter-Trackingeinheit eine Video-Trackingeinheit mit einer separaten Videokamera ist, die mittels Bilderkennung die Position des Kopfes oder der Augen des Betrachters identifiziert und verfolgt.

11. Vorrichtung nach einem der Ansprüche 4 bis 10, **dadurch gekennzeichnet, dass** die Bildverarbeitungseinheit diejenige des Navigationssystems ist oder dieser unmittelbar zugeordnet ist.

12. Vorrichtung nach einem der Ansprüche 1 bis 11, **dadurch gekennzeichnet, dass** sie eine Benutzer-Eingabevorrichtung aufweist, mittels der insbesondere:
- zwischen stereoskopischer und Normalbild-Betrachtungsweise umgeschaltet werden kann; und/oder
- zwischen der Darstellung mit einer oder mehreren Betrachtungszonen umgeschaltet werden kann, und/oder
- Eingaben für eine bild unterstützte Behandlung oder Behandlungsplanung ermöglicht werden, und/oder
- bewegliche Kameras angesteuert werden.

## Claims

1. A device for visually combining patient image data from transillumination and/or tomographic imaging methods and/or object image data with video images, comprising an image display device (1), a camera means (7, 8) and a computer-assisted navigation system which can detect the spatial positions of said image display device (1) and/or said camera device (7, 8) and the spatial positions of a part of the patient's body via tracking means (4) attached thereto, wherein the image display device (1) and the camera device (7, 8) are assigned to each other and formed as a portable unit, **characterised in that** the image display device (1) comprises an auto-stereoscopic monitor (3).

2. The device as set forth in claim 1, **characterised in that** the camera means is a stereoscopic camera means (7, 8).

3. The device as set forth in claim 1 or 2, **characterised in that** the camera means comprises at least two cameras (7, 8) which are arranged on the rear side of the device (1) on the opposite side to the monitor (3), and whose distance from each other is adjusted to a predetermined distance range between the observer and the monitor (3).

4. The device as set forth in claim 3, **characterised in that** the camera means comprises movable cameras (7, 8), in order to set the intersecting region of the fields of vision of the cameras in accordance with the distance between the cameras and the object observed and/or in accordance with the distance between the observer and the monitor (3).

5. The device as set forth in any one of claims 1 to 4, **characterised in that** the monitor (3) is controlled by an image processing unit which spatially assigns and combines the stereoscopic video images and three-dimensionally calculated patient body structures from the transillumination images and/or tomographs, to display a combined stereo image.

6. The device as set forth in any one of claims 1 to 5, **characterised in that** the monitor (3) is an aperture mask or lens monitor having one or more stereo observation zones.

7. The device as set forth in claim 6, **characterised in that** said observation zone(s) are arranged stationary relative to the monitor (3).

8. The device as set forth in claim 6, **characterised in that** an observer tracking unit is assigned to the monitor (3) and the observation zone(s) are tracked to the observer, by the image processing unit displaying an image on the monitor (3) which develops its observation zone(s) at the or each tracked observer location.

9. The device as set forth in claim 8, **characterised in that** said observer tracking unit is a tracking unit of the navigation system or is directly assigned to it.

10. The device as set forth in any one of claims 4 to 8, **characterised in that** the observer tracking unit is a video tracking unit with a separate video camera which identifies and tracks the position of the head or the eyes of the observer by means of image recognition.

11. The device as set forth in any one of claims 4 to 10, **characterised in that** the image processing unit is the image processing unit of the navigation system or is directly assigned to it.

12. The device as set forth in any one of claims 1 to 11, **characterised in that** it comprises a user input device, by means of which in particular:
- it is possible to toggle between a stereoscopic and normal image observation mode; and/or
- it is possible to toggle between displaying with one or more observation zones; and/or
- inputs for image-assisted treatment or treatment planning are enabled; and/or
- movable cameras are controlled.

## Revendications

1. Dispositif pour la combinaison visuelle de données graphiques d'un patient obtenues par un procédé radiographique ou un procédé tomographique ou de données d'images-objets avec des images vidéo, comportant un dispositif d'affichage d'image (1), un dispositif à caméras (7,8) et un système de navigation assisté par ordinateur qui peut relever les positions spatiales du dispositif d'affichage d'image (1) et/ou du dispositif à caméras (7, 8), ainsi que les positions spatiales d'une partie corporelle d'un patient par l'intermédiaire de dispositifs de poursuite (4), le dispositif d'affichage d'image (1) et le dispositif à caméras (7, 8) étant associés l'un à l'autre et réalisés sous la forme d'une unité portable, **caractérisé en ce que** le dispositif d'affichage d'image (1) comporte un moniteur (3) autostéréoscopique.

2. Dispositif selon la revendication 1, **caractérisé en ce que** le dispositif à caméras est un dispositif à caméras stéréoscopique (7, 8).

3. Dispositif selon la revendication 1 ou 2, **caractérisé en ce que** le dispositif à caméras comprend au moins deux caméras (7, 8) qui sont disposées sur la face arrière, opposée au moniteur (3), du dispositif (1), et dont l'écartement est adapté à une plage de distance prédéfinie entre l'observateur et le moniteur (3).

4. Dispositif selon la revendication 3, **caractérisé en ce que** le dispositif à caméras comporte des caméras mobiles (7, 8) afin de régler la zone de recoupement des champs visuels des caméras en accord avec la distance entre les caméras et l'objet observé et/ou en accord avec la distance entre l'observateur et le moniteur (3).

5. Dispositif selon l'une des revendications 1 à 4,
**caractérisé en ce que** le moniteur (3) est commandé par une unité de traitement d'image qui associe dans l'espace et combine les images vidéo stéréoscopiques et les structures corporelles du patient calculées en trois dimensions à partir des images radiographiques ou tomographiques, une image stéréo combinée étant affichée.

6. Dispositif selon l'une des revendications 1 à 5, **caractérisé en ce que** le moniteur (3) est un moniteur à masque perforé ou un moniteur à lentilles avec une zone d'observation stéréo ou avec plusieurs zones d'observation stéréo.

7. Dispositif selon la revendication 6, **caractérisé en ce que** la (les) zone(s) d'observation sont disposées de manière fixe par rapport au moniteur (3).

8. Dispositif selon la revendication 6, **caractérisé en ce qu'**au moniteur (3) est associée une unité de poursuite d'observateur et la (les) zone(s) d'observation suit (suivent) l'observateur **en ce que**, par l'unité de traitement d'image, est affichée sur le moniteur (3) une image qui développe sa (ses) zone(s) d'observation à l'emplacement ou à chaque emplacement poursuivi de l'observateur.

9. Dispositif selon la revendication (8), **caractérisé en ce que** l'unité de poursuite d'observateur est une unité de poursuite du système de navigation ou est directement associée à celle-ci.

10. Dispositif selon l'une des revendications 4 à 8, **caractérisé en ce que** l'unité de poursuite d'observateur est une unité de poursuite vidéo avec une caméra vidéo séparée qui identifie et suit, par reconnaissance d'images, la position de la tête ou des yeux de l'observateur.

11. Dispositif selon l'une des revendications 4 à 10, **caractérisé en ce que** l'unité de traitement d'image est celle du système de navigation ou est directement associée à celle-ci.

12. Dispositif selon l'une des revendications 1 à 11, **caractérisé en ce qu'**il comporte un dispositif de saisie utilisateur, au moyen duquel on peut en particulier :
- commuter entre le mode d'observation stéréoscopique et le mode d'observation d'images normales ; et/ou
- commuter entre la représentation avec une ou plusieurs zones d'observation, et/ou
- il est possible de procéder à des saisies pour un traitement ou un programme de traitement assisté par image, et/ou
- au moyen duquel des caméras mobiles sont commandées.
